(19) Europäisches Patentamt
European Patent Office
Office européen des brevets

(11) **EP 4 721 770 A1**

(12) **EUROPEAN PATENT APPLICATION**

(43) Date of publication:
08.04.2026 Bulletin 2026/15

(21) Application number: 24204797.5

(22) Date of filing: 04.10.2024

(51) International Patent Classification (IPC):
*A61L 27/18* (2006.01)     *A61L 27/34* (2006.01)
*A61L 27/50* (2006.01)     *A61L 27/56* (2006.01)
*A61L 27/58* (2006.01)

(52) Cooperative Patent Classification (CPC):
(C-Sets available)
**A61L 27/56; A61L 27/18; A61L 27/34;
A61L 27/507; A61L 27/58**          (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Technische Universität München, in
Vertretung des Freistaats Bayern
80333 München (DE)**

(72) Inventors:
• **Müller, Kilian
80689 Munich (DE)**
• **Mela, Petra
85748 Garching (DE)**
• **Ahrens, Christina
80339 München (DE)**

(74) Representative: **Lucke, Andreas
Boehmert & Boehmert
Anwaltspartnerschaft mbB
Pettenkoferstrasse 22
80336 München (DE)**

(54) **VASCULAR GRAFT DEVICE AND MANUFACTURING METHOD**

(57)    A vascular graft device (1), comprising a liner (3) comprising a microporous structure made from a first polymer material, wherein the first polymer material is hemocompatible; and a structural scaffold (5) supporting the liner (3), wherein the structural scaffold (5) comprises a macroporous fiber structure (50), made from a second polymer material wherein the second polymer material is biodegradable.

*Fig. 1a*

EP 4 721 770 A1

**(Cont. next page)**

(52) Cooperative Patent Classification (CPC): (Cont.)

C-Sets
**A61L 27/34, C08L 89/06**

**Description**

TECHNICAL FIELD

The present application relates to a vascular graft device. Generally, vascular graft devices are devices that can replace a section of a blood vessel, such as an artery or a vein. A vascular graft device may realize a vascular prosthetic device (also referred to as "vascular prosthesis"). Such devices can be coronary artery by-pass grafts, artificial by-pass grafts, by-pass shunts, arteriovenous grafts, homografts, pulmonary allografts, hemodialysis tubes, and/or pulmonary shunts. The present application further relates to a manufacturing method for a vascular graft device

TECHNICAL BACKGROUND

Autologous grafts (vein or artery segments) are the gold standard in (coronary) by-pass surgeries. However, they are not always available due to previous harvest, anatomical variability, or disease progression.

Clinically available synthetic grafts are conventionally made from polyurethane (PU), expanded polytetrafluoroethylene (ePTFE, GORE-TEX®), or poly(ethylene terephthalate) (Dacron®). For small diameter (< 6mm) grafts they often perform poorly due to surface of thrombogenicity, infection risk, and intimal hyperplasia, which may lead to stenotic failure. Additionally, these grafts are "non-living", and, therefore, lack growth and remodeling capabilities as found in living tissues. A further disadvantage of synthetic grafts is their susceptibility for infection. "living" grafts are less prone to infection.

Alternatively, tissue engineering (TE) small diameter vascular grafts in vitro has been proposed. US 2022/0257371 A1 proposes a soft tissue scaffold for regeneration of blood vessels. The scaffold comprises a first region comprising fibers forming an anisotropic tissue, which is designated to act as a support for the leaflets of a heart valve. The anisotropic tissue is wrapped around a mandrel and then attached to a second set of melt-electrowritten (MEW) fibers arranged approximately parallel relative to one another, forming a tubular scaffold. The second region supports the first region. The fibers shall be arranged such that a spacing between adjacent fibers results in a pore size which allows cellular proliferation. This gap between adjacent fibers shall be 0.1 mm to about 2.0 mm. After being infused with cellular material, such as epithelial cells capable of forming an aortic root, the infused scaffold shall be capable to display mechanical properties similar to native tissue.

Despite the tremendous advances made in the field of TE over the past decade, the translation of in vitro tissue-engineered constructs into the clinics is still limited or has failed, inter alia due to variability issues, scalability challenges, and regulatory burdens. In this context, TE paradigms shifted towards the in situ TE approach, where cell-free constructs are implanted to summon the natural regenerative potential by steering the immune response of the host body. In situ TE circumvents regulatory issues associated with in vitro TE, and as such provides off-the-shelf solutions for implants.

Exemplarily, the Dutch company Xeltis is currently investigating an in situ TE graft as arterio-venous access shunt for hemodialysis in a first-in-human feasibility study. This graft builds on a supramolecular polymeric material platform that is processed via solution electrospinning into microporous tubular grafts. Unfortunately, for these grafts, multiple re-interventions due to stenotic events were reported. Furthermore, solution- or dispersion-electrospinning is often performed using toxic solvents or dispersing medium which is considered disadvantageous for the biocompatibility of the resulting graft.

It is desired to provide a vascular graft device which overcomes the disadvantages of the prior art, in particular with regard to small diameter vessels, namely thrombogenicity and development of intimal hyperplasia, and which displays excellent biocompatibility and is suitable for in situ tissue regeneration, and to provide a method of manufacturing such a vascular graft device.

SUMMARY

The technical problems as indicated herein are solved by the subject matter of the independent claims. Particular examples of the claimed subject matter are given by the dependent claims.

According to a first aspect, a vascular graft device is provided. The vascular graft device may be manufactured according to the further aspect of the present disclosure. The vascular graft device comprises or consists of a liner and a structural scaffold and possibly a hydrogel coating. The mechanical properties of the vascular graft device may substantially be defined by the structural scaffold and the hemocompatibility of the vascular graft device may substantially be provided by the liner and/or a hydrogel coating. The liner comprises a microporous structure, in particular a microporous fiber structure. Alternatively, the liner may comprise or consist of a microporous structure provided by a tubing, such as a silicone tubing. The microporous structure may be made from, in particular may consist of, a first polymer material. The first polymer material may in particular be hemocompatible. The first polymer material may in particular comprise or may consist of a silicone material, such as silicone rubber, polyethylene, acrylic resins, polyurethane, polypropylene, and/or polymethylmethacrylate. Alternatively or additionally, the first polymer material may comprise or may consist of polyglycolide, polylactide, polyhydroxobutyrate, chitosan, hyaluronic acid, and/or a hydrogel. The first polymer material may be a homo-polymer or a co-polymer. In an embodiment the first polymer material includes poly E - caprolactone (PCL), a poly (glycolide - co - trimethylene carbonate - co - caprolactone ) thermopolymer such as Strataprene® from Poly - Med Inc., poly (carbonate urethane ) urea, a poly urethane and / or poly (ester urethane)urea. The first polymer material may include inter alia, but not limited to: Bioresorbable polymers (such as poly lactic acid (PLA), including poly(L-lactide), poly(D-lactide), poly(D,L-lactide), as Well as polyglycolide, polycaprolactone, polydioxanone, poly(trimethylene carbonate), poly(4-hydroxybutyrate), poly(ester amides) (PEA), polyurethanes, poly(trimethylene carbonate), poly(ethylene glycol), poly(vinyl alcohol), polyvinylpyrrolidone, and copolymers thereof such as poly(L-lactide/DL-lactide), poly(L-lactide/D-lactide), poly(L-lactide/glycolide), poly(L-lactide/caprolactone), poly(DL-lactide/glycolide), non bioresorbable materials (such as polypropylene, polyethylene, polyethylene terephthalate, polytetrafluoroethylene, polyaryletherketone, nylon, fluorinated ethylene propylene, polybutester, and silicone, or copolymers thereof), biological components (such as hyaluronan, collagen, gelatin, chitosan, alginate, aloe/pectin, cellulose or other biological materials originating from tissues from either autologous, allergenic or xenogenic origin), or a combination thereof. In particular, the first polymer material may comprise or may consist of poly(2-hydroxyethyl-methacrylate), polycaprolactone, polyethylene glycol, chitosan, and/or hyaluronic acid. Hemocompatibility refers to how well a biomaterial interacts with blood without causing toxicity. In particular, the term hemocompatibility may designate the capability of materials to be exposed to blood, which may be related to thrombogenic and/or thromboembolic complications induced by biomaterial surfaces.

The structural scaffold may support, in particular support and surrounds, the liner, in particular along the entire length of the vascular graft device. In some embodiments, the liner may surround the structural scaffold, in particular along the entire length of the vascular graft device, wherein in particular the structural scaffold supports the liner, in particular along the entire length of the vascular graft device. In some embodiments, the liner may be arranged radially inside of the structural scaffold, wherein in particular the liner forms the luminal surface of the vascular graft device. In some other embodiments, the structural scaffold may be arranged radially inside of the liner, wherein in particular the structural scaffold may form the luminal surface of the vascular graft device. Further alternatively, it is conceivable that the liner is radially sandwiched between two structural scaffolds. The structural scaffold may comprise a macroporous fiber structure. The macroporous fiber structure may be made from a second polymer material. The second polymer material may in particular be biodegradable, preferably bioresorbable. The second polymer material may in particular be polycaprolactone (PCL), polylactic acid, and/or poly(lactic-co-glycolic) acid. Alternatively or additionally, the second polymer material may comprise or may consist of polyglycolide, polylactide, polyhydroxobutyrate, chitosan, hyaluronic acid, and/or a hydrogel. The second polymer material may be a homo-polymer or a co-polymer. In an embodiment the second polymer material includes poly E - caprolactone (PCL), a poly ( glycolide - co - trimethylene carbonate - co - caprolactone ) thermopolymer such as Strataprene® from Poly - Med Inc., poly ( carbonate urethane ) urea, a poly urethane and / or poly ( ester urethane) urea. The second polymer material may include inter alia, but not limited to: Bioresorbable polymers (such as poly lactic acid (PLA), including poly(L-lactide), poly(D-lactide), poly(D,L-lactide), as Well as polyglycolide, polycaprolactone, polydioxanone, poly(trimethylene carbonate), poly(4-hydroxybutyrate), poly(ester amides) (PEA), polyurethanes, poly(trimethylene carbonate), poly(ethylene glycol), poly(vinyl alcohol), polyvinylpyrrolidone, and copolymers thereof such as poly(L-lactide/DL-lactide), poly(L-lactide/D-lactide), poly(L-lactide/glycolide), poly(L-lactide/caprolactone), poly(DL-lactide/glycolide), non bioresorbable materials (such as polypropylene, polyethylene, polyethylene terephthalate, polytetrafluoroethylene, polyaryletherketone, nylon, fluorinated ethylene propylene, polybutester, and silicone, or copolymers thereof), biological components (such as hyaluronan, collagen, gelatin, chitosan, alginate, aloe/pectin, cellulose or other biological materials originating from tissues from either autologous, allergenic or xenogenic origin), or a combination thereof. Biodegradable materials can break down naturally in the body or the environment, typically through biological processes involving microorganisms. The breakdown of biodegradable polymers may depend on a variety of factors comprising the polymer structure, pH and temperature of the environment. The biodegradable polymer material and its breakdown products are preferably non-toxic.

The first polymer material may in particular be different from the second polymer material. The liner and/or the structural scaffold, in particular the entire vascular graft device, may be made in an extrusion process, such as an electrospinning process or, preferably, in a melt electrowriting process. It may be preferred that the first and/or the second polymer material(s) are free of any solvent or dispersion medium. Additionally or alternatively, the liner and/or the structural scaffold, in particular the entire vascular graft device, may be free of any wrapped microporous lining structure. Further additionally or alternatively, the liner and/or the structural scaffold, in particular the entire vascular graft device, may be free of a seam (i.e. a seam connecting one fiber structure to itself or to another fiber structure). It may be preferred that the vascular graft device is free of a stent structure.

The vascular graft device may in particular be tubular. The term tubular is intended to cover devices of varying sizes (e.g., length, thickness, and/or diameter), shapes, and compositions. Such tubular devices are generally characterized as having a central lumen. A tubular vascular graft device can generally be described as having both an inner diameter and an outer diameter. The vascular graft device has a 3-dimensional shape. The tubular shape of the vascular graft device may be substantially cylindrical. In some embodiments, the vascular graft device may comprise one or more bifurcations, for example, the vascular graft device may comprise a main tubular section which splits up into several, in particular two, tubular subsections. The main tubular section may have a larger inner diameter in comparison to a respective inner diameter of its tubular subsections. A vascular graft device comprising one or more bifurcations may be beneficial in some applications, for example in an arteriovenous shunt, in particular to reduce the flow in order to help prevent arterialization of the receiving vein or veins.

In particular, the vascular graft device may have the shape of a right circular cylinder. Alternatively, the vascular graft device may have a generally cylindrical shape having a circular, elliptical or generally rounded cross section. The cross section of the vascular graft device may be constant or may change along the length of the vascular graft device. The cylindrical shape of the vascular graft device may have a constant inner diameter or an inner diameter changing in the lengthwise direction of the vascular graft device. The wall thickness of the vascular graft device may be substantially constant. In particular, the present disclosure may relate to a small-diameter vascular graft device, having an inner diameter of 6 mm or less. The vascular graft device according to the present disclosure may in particular have an inner diameter of less than 40 mm, of less than 25 mm, of less than 10 mm, of less than 8 mm, of less than 6 mm, of less than 4 mm, in particularly of less than 3.5 mm, of less than 3 mm, of less than 2.5 mm, of less than 2 mm, of less than 1.5 mm, or of less than 1 mm. The vascular graft device may have a total wall thickness of 1500 $\mu$m or less, in particular of 1300 $\mu$m or less, more particularly of 1000 $\mu$m or less, for example of 750 mm or less. Common small-diameter vascular graft devices are known to have an inherent thrombogenicity.

A fiber structure may be realized as an isotropic or as an anisotropic fiber structure. The fiber structure may in particular comprise a regular fiber pattern, or, alternatively, an irregular fiber pattern. For example, the fiber pattern may be a diamond pattern defined by two complementary fiber winding angles. The fiber structure comprises one or more fibers. The fiber may have a diameter ranging from about to nm to about 100 $\mu$m. A spacing between adjacent fibers may form pores. The pores may have a size ranging from about 1 $\mu$m to about 5 mm. Macropores may be provided to allow cellular growth on and/or around the vascular graft device. Therefore, the size of the macropores may be determined by the cells intended to be seeded onto/infused into the scaffold and the type of tissue that is intended to be grown on the scaffold.

The terms "microporous" and "macroporous" as used herein shall be understood with regard to the porosity in relation to blood and its constituents. A microporous structure, in particular a microporous fiber structure, may in particular be configured to be impermeable for blood. In other words, a microporous structure may in particular be fluid-tight with regard to blood. A microporous structure may in particular be capable to receive or be partially infiltrated or be partially penetrated by blood cells so as to allow for blood clotting inside of the microporous structure, particularly inside of the liner, of the vascular graft device. In particular, a microporous structure, in particular a microporous fiber structure, may comprise pores no larger than a predetermined threshold level configured to contain blood within the vascular graft device. The liner comprising a microporous structure, in particular a microporous fiber structure, may in particular be configured such that blood is hindered by the liner from exiting from the lumen of the vascular graft device in a radial direction. The pore size of the microporous structure may in particular be less than 30 $\mu$m, more particularly less than 20 $\mu$m or less than 15 $\mu$m. The pore size of the microporous structure may in particular be in a range of 1 $\mu$m to 30 $\mu$m, more particularly in a range of 2 $\mu$m to 20 $\mu$m, or in a range of 3 $\mu$m to 15 $\mu$m. A macroporous fiber structure may in particular be configured to be permeable for blood. Additionally, a macroporous fiber structure may be adapted to allow or to enhance cellular growth and/or to steer cellular orientation. The macroporous scaffold structure may in particular define the mechanical resilience of the vascular graft device. The pore size of the macroporous fiber structure may in particular be more than 40 $\mu$m, more than 60 $\mu$m, more than 80 $\mu$m, more than 100 $\mu$m, more than 200 $\mu$m or more than 500 $\mu$m. The pore size of the macroporous fiber structure may in particular be in a range of 40-2000 $\mu$m, in a range of 100-1500 $\mu$m or in a range of 200-1200 $\mu$m.

According to a further embodiment of a vascular graft device, the macroporous fiber structure is configured according to at least one (first) predetermined structural parameter selected from the list comprising a fiber pattern, a fiber winding angle, a fiber spacing, a fiber diameter and a number of layers (layer number). The skilled person understands that the macroporous fiber structure may be configured according to at least one set of (first) predetermined structural parameters including one or more of a fiber pattern, a fiber winding angle, a fiber spacing, a fiber diameter and a number of layers. The macroporous fiber structure may for example comprise 15 layers of two fibers wound complementary in a diamond pattern and having a fiber diameter of 20 to 25 $\mu$m wound at fiber winding angles of 15°, 22.5°, 30°, 45°, 60° or 75°, or any angle in between, with an inter fiber distance of 750 $\mu$m $\pm$ 50 $\mu$m.

The at least one (first) predetermined structural parameter may be configured to provide the vascular draft device with a desired compliance value or a target compliance range. The term "compliance" may be understood to refer to the radial extensibility of a vessel or of the vascular graft device in response to a luminal pressure wave. Controlling the fiber winding angle and/or at least one other (first) predetermined structural parameter may serve to tune a compliance value of the vascular graft device according to the physiological properties of arteries and veins. Configuring the vascular graft device in accordance with physiological properties has proven to be advantageous, both from a biological and a mechanical perspective. A gross mismatch between physiological properties and the properties of a vascular graft device may increase the risk of intimal hyperplasia (IH) substantially. The compliance of a vascular graft device may be determined in accordance with ISO 7198:2016-08 (Cardiovascular implants and extracorporeal systems - Vascular prostheses - Tubular vascular grafts and vascular patches, 2nd edition 08/2016, ISO/TC 150/SC 2, ICS: 11.040.40). The compliance may in particular be determined by measuring the change in diameter of the vascular graft device in response to cyclic changes in the luminal pressure from 80 to 120 mmHg. The measurement may be performed with the aid of a high-compliance silicone measurement-liner (with a compliance significantly higher than the compliance of the vascular graft device, e.g. $25.7 \pm 3.5$ %(100 mmHg)$^{-1}$). The measurement-liner may be inserted into a test subject (the vascular graft device or its scaffold structure) for measuring the compliance of the respective test subject. A preferred target compliance range may for example be defined as a range of 0 to 4 % (100 mmHg)$^{-1}$ or as a range of 5 to 15 % (100 mmHg)$^{-1}$. A target compliance range may in particular be defined in accordance with the physiological properties of a human vein or of a human artery.

The fiber structure, in particular the macroporous fiber structure and/or the microporous fiber structure, may in particular be shaped as a diamond fiber pattern. A diamond fiber pattern may be realized by a structure having two complementary fiber winding angles. A fiber winding angle may in particular be defined relative to a longitudinal (lengthwise) axis as a mandrel holding the vascular graft device is rotating or the rotary axis of the vascular graft device. The fibers may be directed along a helical path corresponding to the fiber winding angle. The fiber structure may comprise fibers having a linear path along the fiber pattern. It is conceivable, that the individual fibers may alternatively be laid according to a fiber pattern along paths differing from a linear path, for example along a sinusoidal path. Alternatively, patterns with auxetic behavior could be realized. Patterns with auxetic behavior are a type of patterns with a negative Poisson's ratio, wherein an axial elongation causes a transversal elongation. A pattern with auxetic behavior may for example be realized using concave hexagons (with opposing linear sides connected by opposing concave V-shaped sides).

The macroporous fiber structure may have a fiber diameter in a range of 10 $\mu$m to 50 $\mu$m, in particular in a range of 15 $\mu$m to 30 $\mu$m, more particularly in a range of 20 $\mu$m to 25 $\mu$m. In some examples, the fiber diameter forming the macroporous fiber structure may be in a range of $25 \pm 5$ $\mu$m, more particularly in a range of $23 \pm 2.5$ $\mu$m. The macroporous fiber structure may have a substantially constant fiber diameter along the entire length of the vascular graft device or along at least one section of the entire length of the vascular graft device. Alternatively, the macroporous fiber structure may have a first fiber diameter in a first section of the vascular graft device and a second fiber diameter in a second section of the vascular graft device, wherein the first fiber diameter may be different from the second fiber diameter, i.e. may be smaller or larger than the second fiber diameter. A fiber structure having sections with different fiber diameters may in particular comprise a gradient section within which the fiber diameter changes from the first fiber diameter to the second fiber diameter. It may be preferred that a first fiber diameter in a first section of the vascular graft device is substantially equal to a second fiber diameter in a second section of the vascular graft device. A larger fiber diameter may be associated with a decreased compliance of the vascular graft device.

The macroporous fiber structure may have a fiber spacing (or, inversely: a fiber density) in a range of 100 $\mu$m to 1500 $\mu$m, for example 750 $\mu$m $\pm$ 150 $\mu$m. The fiber spacing of the macroporous fiber structure may in particular be at least 5 times as large as its fiber diameter, in particular at least 10 times as large, preferably at least 20 or 30 times as large as its fiber diameter. In particular, the fiber spacing may be 250 $\mu$m $\pm$ 50 $\mu$m, 550 $\mu$m $\pm$ 100 $\mu$m, 750 $\mu$m $\pm$ 150 $\mu$m or 1000 $\mu$m $\pm$ 150 $\mu$m. A larger fiber spacing (or, inversely: a lower fiber density) may be associated with an increased compliance of the vascular graft device.

The macroporous fiber structure may comprise a number of layers of fibers arranged, in particular complementarily, one above another. The macroporous fiber structure may comprise or may consist of 1 to 50 layers, in particular 5 to 30 layers, more particularly 10 to 20 layers, for example 15 layers, of fibers arranged one above another. A larger number of layers may be associated with a decreased compliance of the vascular graft device.

According to a further development, the vascular graft device comprises at least one gradient section extending in the lengthwise direction of the vascular graft device, wherein the at least one predetermined structural parameter, in particular the at least one (first) predetermined structural parameter of the macroporous fiber structure, changes continuously from a first value to a second value along the gradient section. A continuous change of the at least one predetermined structural parameter shall be understood to relate to a gradual rather than to a discrete change, such as a change according to a continuous function, more particularly according to a smooth function.

The vascular graft device may be configured to comprise a gradient section extending between, a first section configured according to a first compliance target range, and a second section configured according to a second compliance target range. The first compliance target range may in particular be in a range of 0 to 4 % (100 mmHg)$^{-1}$. The second compliance target range may in particular be in a range of 5 to 15 % (100 mmHg)$^{-1}$. At least one other (first) predetermined structural parameter may remain constant within the gradient section, in particular the number of layers, the fiber spacing, the fiber diameter and/or the fiber pattern. Alternatively, two or more (first) predetermined structural parameters of the fiber pattern may be changed within the gradient section, in particular the fiber winding angle, the fiber diameter, and/or the fiber pattern. The two or more (first) predetermined structural parameters may be changed within the gradient section in a partially or completely complementary manner (e.g. to enhance compliance), or in a partially opposing manner. A complementary change of multiple (first) predetermined structural parameters may be selected to provide a relatively large change or a property of the vascular graft device within the gradient section. A partially opposite change of multiple (first) predetermined structural parameters may be selected for fine-tuning a property of the vascular graft device, or to improve manufacturing.

The vascular graft device may define a length (i.e. an entire graft length). The first section, the second section and the gradient section of the vascular graft device may each extend over a respective length of the entire graft length. The entire graft length may be at least as large as the inner diameter of the vascular graft device, in particular at least twice as large or at least five times or ten times as large. The first section of the vascular graft device may have a length in arrange of 10% to 40% of the entire graft length. The length of the first section of the vascular graft device may be in a range of 1 mm to 30 mm, in particular in a range of 5 mm to 15 mm. The second section of the vascular graft device may have a length in a range of 10% to 40% of the entire graft length. The length of the second section of the vascular graft device may be in a range of 1 mm to 30 mm, in particular in a range of 5 mm to 15 mm. The first section and the second section may be of substantially equal length. The at least one gradient section of the vascular graft device may have a length in a range of 20% to 80% of the entire graft length. It shall be clear that the respective lengths of the first section, of the second section and of the gradient section may be adapted such that they form the entire graft length together. It may be preferred that the at least one gradient section is longer than the first section and/or the second section.

In some embodiments of the vascular graft device, the vascular graft device is configured to be implanted at a host, in particular at a host (blood) vessel, wherein the at least one (first) predetermined structural parameter may be selected according to a mechanical compliance matching a characteristic mechanical compliance of a predetermined type of host (blood) vessel, such as an arterial host vessel or a venous host vessel.

According to some embodiments of the vascular graft device, the at least one predetermined structural parameter, in particular the at least one first predetermined structural parameter of the macroporous fiber structure, comprises a fiber winding angle in a range of 5° to 80°. The fiber winding angle of the structural scaffold may be referred to as the main fiber winding angle. In particular, the fiber structure is configured to have two complementary fiber winding angles. In some embodiments, the vascular graft device comprises at least a first section (arterial section) having a fiber winding angle in a range of 5° to 35°, in particular in a range of 15° to 30°. Alternatively or additionally, the vascular graft device comprises at least a second section (venous section) having a fiber winding angle in a range of 40° to 80°, in particular in a range of 45° to 75°. The vascular graft device may additionally comprise at least one gradient section (intermediate section), which maybe arranged between said first (particularly arterial) section and said second (particularly venous) section, within which the fiber winding angle changes, in particular continuously, from a first value to a second value larger than the first value. Said value may correspond to said range of fiber angles of the first section, for example 35°. Said second value may correspond to said range of fiber angles of the second section, for example 45°.

According to some embodiments, the microporous fiber structure may be configured according to at least one (second) predetermined structural parameter selected from the list comprising a fiber pattern, a fiber winding angle, a fiber spacing, a fiber diameter and a number of layers . The microporous fiber structure may in particular be configured according to a set of (second) predetermined structural parameters. It may be preferred if the (second) predetermined structural parameter or a set thereof is constant for the entire length of the vascular graft device. Alternatively, the at least one (second) predetermined structural parameter may change, in particular within the gradient section.

It may be preferred if the at least one (second) predetermined structural parameter of the microporous fiber structure differs from the at least one (first) predetermined structural parameter of the macroporous fiber structure. In particular, the at least one (second) predetermined structural parameter of the microporous fiber structure may be configured such that the compliance of the microporous fiber structure is at least as large as, in particular larger than, the compliance of the macroporous fiber structure. The microporous fiber structure may for example have a second fiber diameter being smaller than a first fiber diameter of the macroporous fiber structure. The microporous fiber structure may have a second fiber diameter in a range of 1 $\mu$m to 20 $\mu$m, in particular in a range of 3 $\mu$m to 15 $\mu$m, more particularly in a range of 7 $\mu$m to 10 $\mu$m. In some embodiments, the second fiber diameter may be 9 $\pm$ 1 $\mu$m, in particular 8.5 $\pm$ 0.5 $\mu$m or 9 $\mu$m $\pm$ 0.5 $\mu$m.

Unless explicitly stated otherwise, the disclosure regarding the (first) predetermined structural parameter of the macroporous fiber structure may apply for the (second) predetermined structural parameter of the microporous fiber structure, and vice-versa.

The fiber winding angle (liner winding angle) of the liner may be in a range of 15° to 75°. It may be preferred that the fiber winding angle of the liner is approximately the same as the main fiber winding angle (main winding angle). The liner winding angle may in particular be configured to deviate from the main winding angle. The liner winding angle may in particular be in the same range of angles as the main winding angle. It may be preferred if the liner comprises several layers having different fiber winding angles. The fiber winding angle of at least one of the layers of the liner may be shifted in a range of $\pm$ 25°, in particular in a range of $\pm$ 15°, more particularly in a range of $\pm$ 5°, relative to the main fiber winding angle. The liner may comprise or may consist of a number of layers in a range from 2 to 20, in particular in a range from 3 to 15, more particularly in a range from 4 to 10. The liner may in particular comprise an innermost layer configured to come into contact with the host's / patient's blood. The innermost layer of the liner may in particular be optimized for hemocompatibility such as to avoid thrombogenic effects. Neither the innermost layer thereof nor the liner in total, however, need to be optimized with respect to mechanical characteristics. In particular, the liner may comprise a microporous fiber structure comprising five stacks of parallel fiber sets. Fibers in a respective set may be configured to have a fiber distance in a range of 50 $\mu$m to 500 $\mu$m, in particular in a range of 100 $\mu$m to 250 $\mu$m, for example 200 $\mu$m. The fiber distance may be the same in multiple sets of the same liner. The liner may have a microporous fiber pattern having a pore size of for example 7.9 $\pm$ 5 $\mu$m. The pore size may be determined by adapting the fiber spacing or the number of fiber sets. The geometrical characteristics of the microporous structure, in particular of the microporous fiber structure, (which may be referred to as microporous scaffold) may be configured such that its resulting compliance is significantly higher than the compliance of the (macroporous) structural scaffold, particularly such that the (macroporous) structural scaffold dominates or defines the mechanical characteristics of the vascular graft device.

In some embodiments, the vascular graft device may further comprise a hydrogel coating, in particular a gelatine coating. This coating may cover the outer surface and/or the luminal surface of the vascular graft device. The hydrogel coating may be arranged, in particular only, on the innermost surface of the vascular graft device, in particular of the liner. Optionally, the liner may be infiltrated by said hydrogel coating. Alternatively or additionally, the hydrogel coating may be arranged, in particular only, on the outer surface of the vascular graft device, in particular of the structural scaffold. Optionally, the structural scaffold may be infiltrated by said hydrogel coating. The hydrogel coating may infiltrate and/or may cover the entire vascular graft device. The vascular graft device may in particular consist of the liner, the structural scaffold and the hydrogel coating. Hydrogel, such as gelatine, may be non-thrombogenic and/or may be configured to provide effective cell adhesion sites. Gelatine may support the formation of endothelium (promote endothelialization). Hydrogels generally have poor mechanical properties, however, in the present description, the mechanical properties of the vascular graft device are provided by the structural scaffold which supports the liner and the hydrogel coating. The hydrogel coating may be chosen such that it does not significantly influence the compliance of the vascular graft device. It could be demonstrated that blood platelet adhesion and activation was drastically reduced in the vascular graft device comprising a gelatine coating in comparison to conventional vascular grafts. The hydrogel/gelatine coating may in particular be functionalized, particularly with anticoagulants such as heparin in order to further improve hemocompatibility. The vascular graft device may be provided with a hydrogel coating, for example a soft gelatine matrix, by dip-coating. The gelatinous matrix preferably does not interfere with the compliance of the vascular graft device. The vascular graft device comprising a hydrogel coating may have a total wall thickness of 1300 $\pm$ 100 $\mu$m.

According to a further aspect, a method of manufacturing a vascular graft device is disclosed. The method may in particular be suitable for manufacturing a vascular graft device according to the first aspect of the present disclosure. The method comprises providing a mandrel, preferably a cylindrical mandrel, in particular a mandrel having a diameter no larger than 40 mm, or no larger than 25 mm, in particular no larger than 10 mm. The mandrel may in particular be configured to have a diameter no larger than 8 mm, more particularly no larger than 6 mm, or no larger than 4 mm. Alternatively, the mandrel may have a generally cylindrical shape having a circular, elliptical or generally rounded cross section. The cross section of the mandrel may be constant or may change along the axial length of the mandrel. The cylindrical shape of the mandrel may have a constant diameter or a changing diameter.

The method may further comprise providing, in particular forming, a liner on the mandrel. The forming of the liner comprises forming a microporous structure, in particular a microporous fiber structure. The forming of the liner may be performed using a hemocompatible first polymer material. A microporous fiber structure may be created in a (first) extrusion process, preferably in a melt electrowriting (MEW) process. Melt electrowriting may be configured to create stacked layers of polymer microfibers along predefined pathways into multilayered architectures resulting in precisely defined porous scaffolds. Melt electrowriting may be performed to create a liner or lining layer configured to realize or act as a precursor for vascular endothelium. Vascular endothelium may comprise a (mono-)layer of endothelial cells and/or may constitute the inner cellular lining of arteries, veins and capillaries and therefore is in direct contact with the components of blood.

The method may further comprise providing, in particular forming, a structural scaffold supporting, in particular supporting and surrounding, the liner. Alternatively, the method may comprise providing, in particular forming, the liner surrounding the structural scaffold.. In some embodiments, the liner may be arranged radially inside of the structural scaffold, wherein in particular the liner forms the luminal surface of the vascular graft device. In some other embodiments, the structural scaffold may be arranged radially inside of the liner, wherein in particular the structural scaffold may form the luminal surface of the vascular graft device. Further alternatively, it is conceivable that the liner is radially sandwiched between two structural scaffolds. Forming the structural scaffold comprises forming a macroporous fiber structure. The forming of the structural scaffold may be performed using a biodegradable second polymer material. The macroporous fiber structure may in particular be created in a (second) extrusion process, preferably in a melt electrowriting process.

The microporous structure, in particular the microporous fiber structure, and the macroporous fiber structure may in particular be created without the use of a solvent or dispersion medium (i.e. by another method than electrospinning). The method of manufacturing a vascular graft device may in particular be performed without connecting the liner and the structural scaffold to a stent. A stent may be a structure, in particular a bistable structure, having a first, contracted, state, and a second, dilated state. Additionally or alternatively, the method of manufacturing a vascular graft device may not comprise wrapping at least one sheet-like fiber structure around the mandrel. Further additionally or alternatively, the method of manufacturing a vascular graft device may not comprise forming a seam connecting one fiber structure to itself or to another fiber structure.

In some embodiments of the method of manufacturing a vascular graft device, the macroporous fiber structure is formed according to at least one (first) predetermined structural parameter or a set of (first) predetermined structural parameters selected from the list comprising a fiber pattern, a fiber winding angle, a fiber spacing, a fiber diameter and a number of layers.

According to some further developments of the method of manufacturing a vascular graft device, the at least one (first) predetermined structural parameter, such as the fiber winding angle, may be changed continuously from a first value to a second value along a lengthwise direction of the vascular graft device during forming of the macroporous fiber structure such that the vascular graft device is provided with at least one gradient section extending in the lengthwise direction of the vascular graft device. Additionally, the method may be configured such that at least one other (first) predetermined structural parameter remains constant as said (first) predetermined structural parameter is changed within the gradient section. The constant other (first) predetermined structural parameter may for example be a fiber diameter, a number of layers and/or a fiber pattern.

Alternatively or additionally, in some embodiments of the method of manufacturing a vascular graft device, the at least one (first) predetermined structural parameter, in particular for forming the macroscopic fiber structure, is selected according to a mechanical compliance matching a characteristic mechanical compliance of a predetermined type of host (blood) vessel, such as an arterial host vessel or a venous host vessel.

In some embodiments of the method of manufacturing a vascular graft device, the microporous structure, in particular the microporous fiber structure, is formed according to at least one (second) predetermined structural parameter or set of (second) predefined structural parameters selected from the list comprising a fiber pattern, a fiber winding angle, a fiber spacing, a fiber diameter and a number of layers. In particular, the at least one (second) predetermined structural parameter may be constant along the length of the vascular graft device.

According to some embodiments of the method of manufacturing a vascular graft device, the at least one (second) predetermined structural parameter of the microporous fiber structure differs from the at least one (first) predetermined structural parameter of the macroporous fiber structure.

In particular, the method of manufacturing a vascular graft device as described hereinabove may further comprise coating the vascular graft device with a hydrogel (matrix), in particular with (a) gelatine (matrix). The coating, in particular the gelatine matrix, may in particular be functionalized, particularly with anticoagulants such as heparin in order to further improve hemocompatibility. The vascular graft device may be provided with a hydrogel, for example a soft gelatine matrix, by dip-coating.

Optionally, the method of manufacturing a vascular graft device may be performed to provide a vascular graft device comprising one or more bifurcations. For example, a vascular graft device may be manufactured comprising a main tubular section and several, in particular two, tubular subsections, wherein the main tubular section splits up into the tubular subsections. A mandrel used for manufacturing such a bifurcated vascular graft device may in particular be shaped accordingly. For example, in case that the bifurcated vascular graft device comprises two tubular subsections, the mandrel used for manufacturing said bifurcated vascular graft device may be Y-shaped.

BRIEF DESCRIPTION OF THE DRAWINGS

FIG. 1a       shows an example of a vascular graft device according to the first aspect formed on a mandrel by melt electrowriting.

FIG. 1b       is a schematic illustration of a vascular graft device according to the first aspect comprising a microporous liner and a macroporous scaffold.

Fig. 2        is a detailed view of the macroporous fiber structure surrounding the microporous fiber structure of the vascular graft device shown in Fig. 1a.

Figs. 3a - 3f   show different examples of vascular graft devices according to the first aspect having macroporous fiber structures with different predetermined structural parameters (e.g. fiber winding angles).

Fig. 4        shows a diagram of the compliance values of the different vascular graft devices of Figs. 3a - 3f and of a silicone liner.

Fig. 5      shows a diagram of the compliance values of different vascular graft devices according to the first aspect having one same first predetermined structural parameter (fiber winding angle) and another differing first predefined structural parameter (e.g. fiber spacing).

Fig. 6a     shows a schematic illustration of another example of a vascular graft device according to the first aspect comprising a gradient section.

Fig. 6b     illustrates a schematic diagram of the fiber winding angle of the fiber structure of the vascular graft device of Fig. 6a.

Fig. 6c     shows an exemplary vascular graft device according to the first aspect comprising a gradient section.

Fig. 6d     shows detailed views of different sections of the vascular graft device of Fig. 6c.

Fig. 6e     shows a microporous fiber structure for the vascular graft device of Fig. 6c comprising a gradient section.

Fig. 6f     shows a macroporous fiber structure for the vascular graft device of Fig. 6c comprising a gradient section.

Fig. 6g     shows the vascular graft device of Fig. 6c coated with gelatine.

Fig. 6h     shows a schematic illustration of an implanted arteriovenous graft (AVG).

Fig. 7      shows a schematic diagram of the compliance values of different vascular grafts according to the first aspect in comparison to compliance values of their structural scaffold alone (i.e. without a liner).

Fig. 8      shows a schematic diagram of diameter reduction values of an exemplary vascular graft device according to the first aspect depending on a bending radius.

Fig. 9      shows a schematic overview of a fabrication process for a vascular graft device according to the further aspect.

DETAILED DESCRIPTION

In the following, some particular examples are described in connection with the accompanying drawings. Same reference signs may indicate same or at least similar features. Any of the above described features and the respective technical effects may apply accordingly to the corresponding features described below in connection with the drawings.

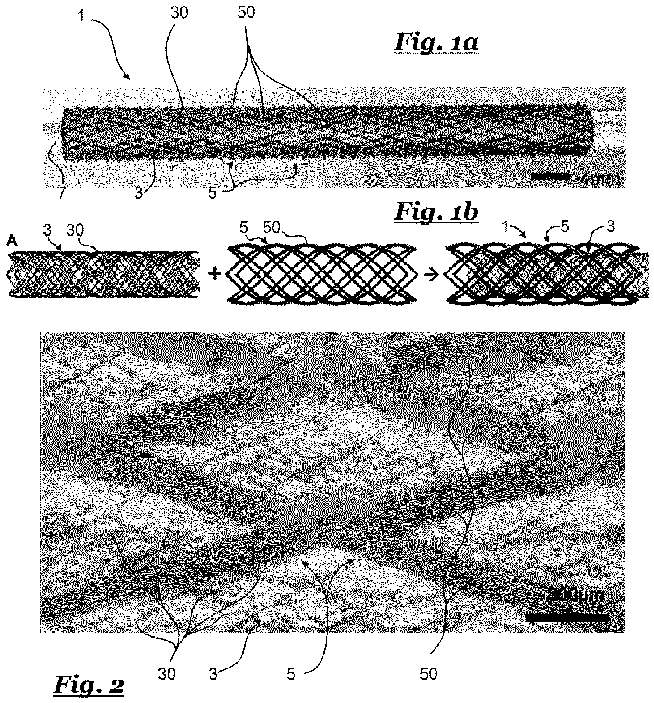

FIG. 1a shows an example of a vascular graft device 1 according to the first aspect formed on a mandrel 7 by melt electrowriting. FIG. 1b is a schematic illustration of a vascular graft device 1 comprising a microporous liner 3 and a macroporous structural scaffold 5. Fig. 2 is a detailed view of the macroporous fiber structure 50 surrounding the microporous fiber structure 30 of the vascular graft device 1 shown in Fig. 1a.

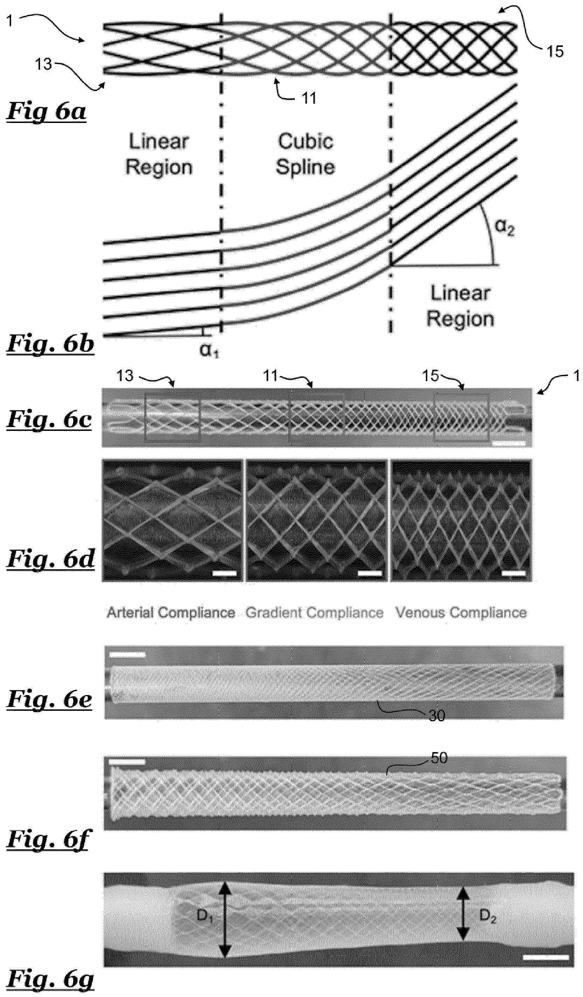

The vascular graft device 1 comprises a liner 3 (luminal layer) composed of a hemocompatible material and a structural scaffold 5 for surrounding and supporting the liner 3 and composed of a melt-electrowritten fiber architecture. In order to form the structural scaffold 5, polycaprolactone fibers are deposited in a MEW process onto the cylindrical mandrel 7. The fibers may in particular be deposited according to two complementary fiber winding angles so as to form an intertwined multi-helix (Figs. 1b, 6a). Linear fiber paths may be connected with circular fiber path segments, especially at the ends of the vascular graft device 1, as shown in Fig. 6c, obtained in a single uninterrupted toolpath considered advantageous for a continuous fiber deposition. The mandrel 7 of the illustrated example has a constant mandrel diameter of 4 mm. Using the mandrel, 7 the resulting vascular graft device 1 is realized as a tubular microporous fiber structure with a diamond fiber pattern. The fiber winding angle $\alpha$ of the fibers with respect to the longitudinal axis of the mandrel 7 may be configured to control the compliance characteristic of the resulting vascular graft device 1.

A positive value of the fiber winding angle $\alpha$ may be indicative of a spiral structure oriented in a right-hand-side with respect to the longitudinal axis of the mandrel 7. A negative value of the fiber winding angle $\alpha$ may be indicative of a spiral structure oriented in a left-hand-side with respect to the longitudinal axis of the mandrel 7. In a vascular graft device 1 which comprises two complementary fiber winding angles, the angle of the right-hand-side spiral and the angle of the left-hand-side spiral may be the same or substantially the same. I.e., the absolute value of the angle of the fibers rotating clockwise (right-hand-side) may be equal to the absolute value of the angle of the fibers rotating counterclockwise (left-hand-side). Alternatively, in a vascular graft device 1 which comprises a pair of two opposing fiber winding angles, the angle of the fibers rotating clockwise (right-hand-side) may differ from the angle of the fibers rotating counterclockwise (left-hand-side), wherein the absolute values of the right-hand-side angle and the left-hand-side angle of the pair may be configured to differ by no more than 15°, in particular no more than 10°, preferably no more than 5°.

It is conceivable that the microporous fiber structure 30 and/or the macroporous fiber structure 50 or at least one or more layers thereof may comprise a fiber pattern which comprises a pattern exclusively revolving either in the right-hand-side or in the left-hand-side. For example the microporous fiber structure 30 may comprise one, two or more layers which do not comprise fiber pattern defined by two complementary fiber winding angles. The fibers of such a layer may revolve exclusively clockwise or exclusively counterclockwise. The microporous fiber structure 30 and/or the macroporous fiber structure 50 may comprise two or more layers arranged directly on top of each other wherein a first layer comprises a fiber pattern revolving exclusively in the clockwise direction and the second layer comprises a fiber pattern revolving exclusively in the counterclockwise direction.

The structural scaffold 5 may be fabricated by precisely depositing polycaprolactone (PCL) microfibers onto the mandrel 7 carrying the liner 3. For creating the structural scaffold 5, multiple layers, such as 15 layers, of fibers with a predetermined fiber diameter of, for example $24.2 \pm 1.6$ $\mu$m, may be stacked. The fibers of the structural scaffold 5 form a macroporous fiber structure 50.

The macroporous fiber structure 50 may have a predetermined main fiber winding angle of for example $\pm 45°$ (or e.g. $\pm 30°$, $\pm 40°$, $\pm 50°$, $\pm 60°$). In these examples, the "$\pm$" is indicative of a fiber pattern defined by two complementary fiber winding angles. The macroporous fiber structure 50 was created on top of the microporous fiber structure 30, which was melt-electrowritten first, to form the vascular graft device's 1 luminal side. Thus, the structural scaffold 5 is created surrounding the liner 3. To this end, large diameter fibers of the macroporous fiber structure 50 may be printed onto small diameter fibers of the microporous fiber structure 30 in a MEW process.

The liner 3 may be fabricated prior to the structural scaffold 5 by precisely depositing microfibers onto the mandrel 7. For creating the liner 3, multiple layers, such as 5 layers, of fibers with a predetermined fiber diameter of, for example $9 \pm 1$ $\mu$m, may be stacked. The fibers of the liner 3 form a microporous fiber structure 30. The microporous fiber structure 30 may comprise five stacks of parallel fiber sets (200 $\mu$m inter fiber distance) shifted $\pm 50$ and $\pm 15°$ relative to the main fiber winding angle of the macroporous fiber structure 50.

The vascular graft device 1 and its constituents may be imaged using a digital light microscope (VHX-5000, Keyence, Germany). The first/second predetermined structural parameters such as the fiber diameter may be determined via a built-in software of the microscope. Pore sizes may be evaluated using light microscopy images, in particular by fitting circles into the pores using MATLAB. The diameter of such fitted circles may be used to define an average pore diameter.

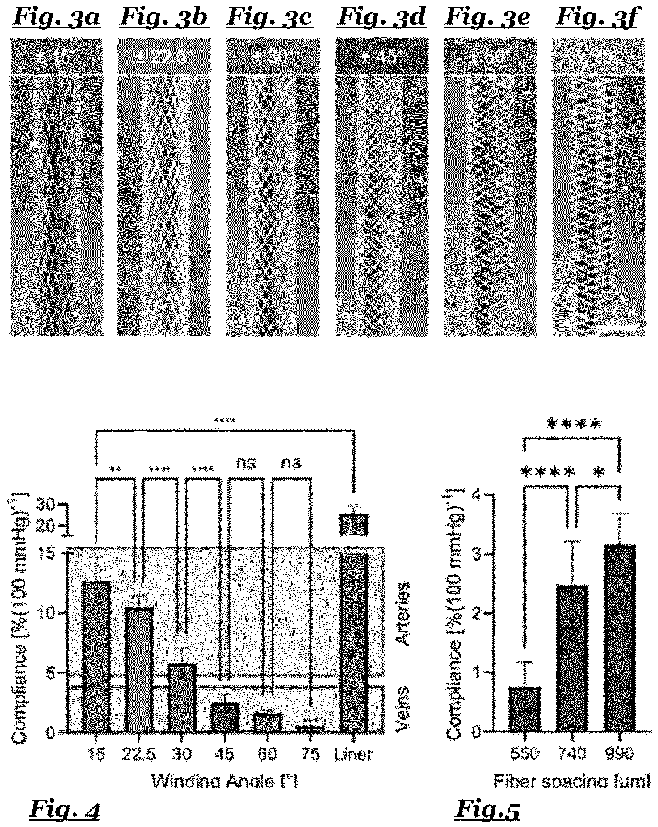

Figs. 3a - 3f show different examples of vascular graft devices 1 having macroporous fiber structures with different first predetermined structural parameters (e.g. fiber winding angle).

In accordance with ISO 7198:2016, the compliance of the different vascular graft devices 1 shown in Figs. 3a to 3f may be determined by measuring a change in diameter in response to cyclic changes in the luminal pressure from 80 to 120 mmHg which may be created by inflating a high-compliance silicone liner ($25.7 \pm 3.5$ %(100 mmHg)$^{-1}$) inside the scaffold. The embodiment of Fig. 3a has a main fiber winding angle of $\pm 15°$. The embodiment of Fig. 3b has a main fiber winding angle of $\pm 22.5°$. The embodiment of Fig. 3c has a main fiber winding angle of $\pm 30°$. The embodiment of Fig. 3d has a main fiber winding angle of $\pm 45°$. The embodiment of Fig. 3e has a main fiber winding angle of $\pm 60°$. The embodiment of Fig. 3f has a main fiber winding angle of $\pm 75°$.

As shown in Fig. 4, different compliances ranging from $12.7 \pm 2.0$ %(100 mmHg)$^{-1}$ for the $\pm 15°$ vascular graft device 1 (Fig. 3a) down to $0.6 \pm 0.4$ %(100 mmHg)$^{-1}$ for the vascular graft device 1 as shown in Fig. 3f with $\pm 75°$ main fiber winding angle were obtained. By controlling at least one first predetermined structural parameter (in this example the main fiber winding angle), the mechanical properties of the vascular graft device can be tuned to cover the range of compliances of native human arteries (4.7 - 17.0 %(100 mmHg)$^{-1}$) and veins (0.7 - 3.7 %(100 mmHg)$^{-1}$). This is a substantial advantage in comparison to conventional stiff synthetic grafts made of polytetrafluoroethylene (PTFE) (0.2 - 0.9 %(100 mmHg)$^{-1}$) or Dacron (0.8 - 1.9 %(100 mmHg)$^{-1}$).

Fig. 5 shows a diagram of the compliance values of the different vascular graft devices having a same first predetermined structural parameter (fiber winding angle) and another differing first predetermined structural parameter (e.g. fiber spacing). Varying the distance between parallel fibers may be used as an alternative or an additional option for tuning the mechanical properties of the vascular graft device 1. Further alternatively or additionally, the fiber diameter and/or the number of layers of fibers stacked above one another in the fiber structure may be modified as a first predefined structural parameter in order to control the mechanical properties of the vascular graft device 1.

Fig. 6a shows a schematic illustration of another example of a vascular graft device 1 comprising a gradient section 11. Fig. 6b illustrates a schematic diagram of the fiber winding angle of the fiber structure of Fig. 6a. Fig. 6c shows an exemplary vascular graft device 1 comprising a gradient section 11. Fig. 6d shows detailed views of different sections of the vascular graft device 1 of Fig. 6c. Fig. 6e shows the microporous fiber structure 30 of the vascular graft device 1 of Fig. 6c comprising a gradient section 11. Fig. 6f shows the macroporous fiber structure 50 of the vascular graft device 1 of Fig. 6c comprising a gradient section 11. Fig. 6g shows the vascular graft device 1 of Fig. 6c coated with gelatine.

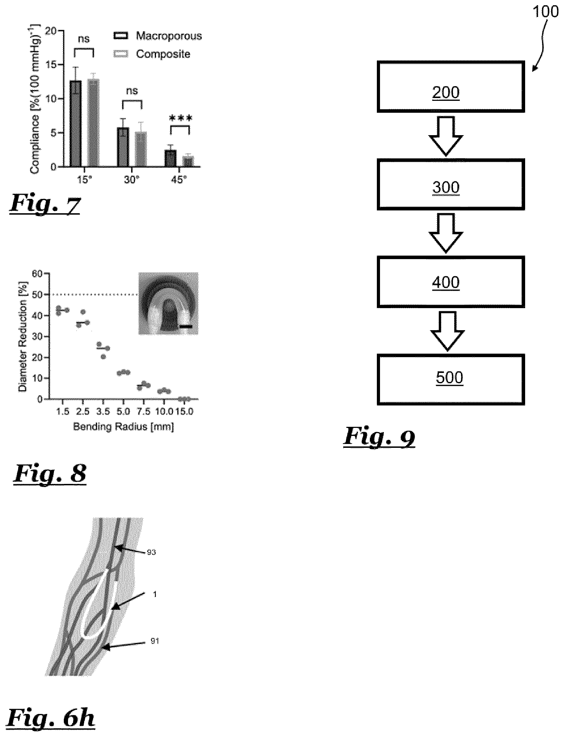

Fig. 6h shows a schematic illustration of an implanted arteriovenous graft (AVG). An AVG may for example be employed as a shunt for hemodialysis such as indicated in Fig. 6h. The vascular graft device 1 indicated therein is implanted as a shunt between a vein 91 and an artery 93. The vascular graft device 1 is able to withstand cannulation, in particular with a 16G needle on a graft with inner pressure of 120 mmHg.

A first section 13 of the vascular graft device 1 having a relatively large compliance may be configured to be connected to the artery 93. A second section 15 of the vascular graft device 1 may be configured to be connected to the vein 91. The first section 13 and the second section 15 may have different, constant or substantially constant, first predetermined structural parameters. As indicated in Figs. 6a and 6b, the first section 13 may have a relatively small fiber winding angle $\alpha_1$, of, for example, 15°, and the second section 15 may have a relatively large fiber winding angle $\alpha_2$, of, for example, 45°. Along the gradient section 11 between the first section 13 and the second section 15, the fiber winding angle may slowly and continuously change from the first winding angle $\alpha_1$ to the second winding angle $\alpha_2$.

The fiber spacing between adjacent parallel fibers in the lengthwise direction of the vascular graft device 1 may be constant. In particular, the same or substantially the same fiber spacing may be defined between adjacent fibers in the first section 13, the second section 15 and/or the gradient section 11. In this way, mechanical properties, such as the compliance characteristics, may smoothly transition from one side (the first section 13) to the other side (the second section 15) of the vascular graft device 1. This may help to obtain a vascular graft device 1 having opposite ends (two sections) which display different mechanical properties, preferably in accordance with those of the (different) vessels to which the vascular graft device 1 is to be attached.

The vascular graft device 1 defines an entire graft length. The first section 13, the second section 15 and the gradient section 11 of the vascular graft device 1 may each extend over a respective length of the entire graft length. The entire graft length may be several cm long, for example 10 cm. The first section 13 and the second section 15 of the vascular graft device 1 have a length of approximately 25% of the entire graft length, respectively. The first section 13 and the second section 15 are of substantially equal length. The gradient section 11 of the vascular graft device 1 may have a length of approximately 50% of the entire graft length.

It may be advantageous that the distance between parallel fibers becomes smaller as the fiber winding angle becomes larger (for example for fitting the mechanical properties of the vascular graft device 1 to a venous vessel with less compliance (as compared to an arterial vessel)), in particular to keep the vascular graft device's 1 circumference constant. This may be beneficial for the compliance gradient, further supporting the compliance difference between venous and arterial regions.

Figs. 6c and 6d show the different sections 11, 13, 15 of the vascular graft device 1 in detail. The first section 13 may be configured to provide an arterial compliance. The fiber winding angle in the first section 13 may be set in a range of 10° to 35°, for example as a first angle $\alpha_1$ of around 15° . The second section 15 may be configured to provide venous compliance. The fiber winding angle in the second section 15 may be set in a range of 40° to 80°. For example, the second fiber winding angle $\alpha_2$ may be set to around 45°.

Figs. 6e and 6f show the microporous fiber structure 30 of the liner 3 and the macroporous fiber structure 50 of the scaffold structure 5 of the vascular graft device 1 shown in Fig. 6g, both comprising a gradient section 11 between a respective first and second section 13, 15 having at least one different first/second predetermined structural parameter. Fig. 6g shows the vascular graft device 1 in a pressurized state in which the first section 13, which is more compliant, is expanded to a larger first graft diameter D1 in comparison to a smaller second graft diameter D2 in the second section 15 which is less compliant.

Fig. 7 shows a schematic diagram of the compliance values of different vascular graft devices in comparison to compliance values of their respective structural scaffold 5 alone. As can be seen, the mechanical properties, in particular the compliance, is essentially determined by the support scaffold 5.

Fig. 8 shows a schematic diagram of diameter reduction values of an exemplary vascular graft device 1 depending on a bending radius. To evaluate the kinking behavior, vascular graft devices 1 may be pressurized with 100 mmHg and bent around a template featuring radii from 1.5 mm to 15 mm. The reduction in diameter was quantified (where Do is the vascular graft device's 1 diameter in relaxed configuration and D1 is the vascular graft device's 1 diameter while undergoing bending):

$$\Delta D = 100\% \times \frac{(D_0 - D_1)}{D_0}$$

200 $\mu$l whole blood was diluted in 10 ml 0.9 % saline solution. The samples ( $\simeq$ 210 mm$^2$ surface area, high density PE (Raumedic, Germany) and ePTFE (GORE-TEX Cardiovascular Patch, W. L. Gore & Associates, USA) as reference materials) may be incubated in 700 µl diluted blood under gentle agitation at 37 °C for 2 h. As positive control (PC) and negative control (NC), whole blood may be incubated with only distilled water or saline solution, respectively. After centrifugation, the absorbance values of the supernatants were measured at 545 nm with a spectrophotometer (Spark, Tecan, Switzerland). Hemolysis rate was calculated using the following formula according to ASTM F 756:2017:

$$\% \; Hemolysis \; = \frac{Absorbance \; of \; test \; sample - Absorbance \; of \; negative \; control}{Absorbance \; of \; positive \; control - Absorbance \; of \; negative \; control} \times 100$$

MEW may in particular be performed on a system comprising two linear mechanical-bearing screw-driven stages (PRO115SL, Aerotech, Germany) for the lengthwise and crosswise direction and a direct-drive rotary stage (ADRS100, Aerotech, Germany) for rotation in the circumferential direction of the mandrel 7. The driven stages may be controlled by Automation1 Studio (Aerotech, Germany) using Gcode. A MEW print head may be mounted on the lengthwise or crosswise-axis. The MEW print head may comprise a syringe (3cc Optimum, Nordson EFD, USA) equipped with a 23 G needle (Nordson EFD, USA) protruding about 1 mm from the print head. Both the syringe and the needle may be heated via a temperature control unit (MIUE04003F/MIUE06012D and MI8844820G, Hotset, Germany). Polymer extrusion may be driven by pressurized air applied to the syringe via an electro-pneumatic controller (ITV1050-31F1CL3, SMC, Japan). Fiber formation may be initiated by applying a positive electrical potential (MPL 200-30000 POS, FuG Elektronik, Germany) to the needle while the cylindrical metal collector (4 mm diameter mandrel 7) is electrically grounded.

Alternatively, in particular for fabricating a vascular graft device 1 with bifurcations (main graft splitting up into two or more daughter vessels) via melt electrowriting, a 5-axis kinematic system may be used. A 5-axis platform may be based based on a cartesian FFF system (Zaribo MK3s, Zaribo, Germany). A MEW print head may be mounted to an X-axis carriage, in particular via an adapter. The 5-axis kinematic system may be configured to have two rotating axes (U, V) in addition to three translating axes (X, Y, Z). Optionally, a slip ring (775, Adafruit, USA) may be employed between the U- and the V-axis. Such a slip ring may connect the electrical ground of the high voltage supply (MPL 200-30000, FuG Elektronik, Germany) to the collector mount (mandrel 7) on the V-axis. Light barrier end-stops (DZ0421, DollaTek, People's Republic of China) may be provided for contact-free homing to each of the axes. The 5-axis kinematic system may be controlled, in particular using RepRap firmware version 3.1.1 via a Duet 2 (Duet3D, United Kingdom). Maximum speed and acceleration may be configured, in particular in the firmware, to be: X-axis: 12000 mm min$^{-1}$, 1000 mm s$^{-2}$; Y-axis: 12000 mm min$^{-1}$, 1000 mm s$^{-2}$; Z-axis: 2000 mm min$^{-1}$, 400 mm s$^{-2}$; U-axis: 5000 mm min$^{-1}$, 1000 mm s$^{-2}$; V-axis: 12000 mm min$^{-1}$, 1000 mm s$^{-2}$. The repeatability of motion commands per axis was may be determined by contacting a dial gauge (Hahn & Kolb, Germany) mounted on the V-axis. All axes may be homed. Optionally, all axes may, in particular after being homed, be moved to their respective minimum and/or maximum position. The print head may be moved to the position of a dial gauge, wherein in particular deviations from a predefined target may be recorded.

The MEW print head of the 5-axis kinematic system and/or of the system comprising two linear mechanical-bearing screw-driven stages may comprise a hollow Shapal ceramic cylinder (Schröder Spezialglas, Germany) into which a syringe (3cc Optimum, Nordson EFD, USA) containing polymer granulates can be inserted. The print head main body may be fabricated via a Form 3BL (Formlabs, USA) from the resin High Temp V2 (Formlabs, USA). Preferably following manufacturer parameters including washing and post-curing (Form Wash, Form Cure, Formlabs, USA). Heating cartridges (hotrod MIUE04003F, Hotset, Germany) and a thermosensor (8600210, Hotset, Germany) may be fitted into the ceramic cylinder and controlled via the Duet board to heat the polymer inside the syringe. A 23 G needle (Nordson EFD, USA) may be connected to the syringe. This needle may protrude, for example by 25 mm, from the print head main body. The needle may be separately heated, for example via one or two ring heaters. Such heaters may consist of an inner layer of Kapton film (CMC 70849, cmc Klebetechnik, Germany) shielding a coiled resistive heating wire (Cr20Ni80, AWG24, 5.551 Ohm, sourcing map, People's Republic of China) and/or a NTC 3950 100 K thermosensor from the high voltage applied to the needle. An outer layer of Kapton film and/or a shrinking tube as the outermost layer may provide mechanical integrity to the heaters. In between the two heating elements, the high voltage may be applied to the needle via an aluminum piece that may be connected to a precision power supply (MPL 200-30000, FuG Elektronik GmbH, Germany). The ground of the power supply may be connected to the collector. The syringe may be connected to pressurized air controlled via an electropneumatic regulator (ITV 1050-31F1CL3, SMC, Japan).

Medical grade poly(ε-caprolactone) (Purasorb PC12, Lot# 2004002576, Corbion, Netherlands) may be melted at 75 °C in the syringe and extruded via the needle at 85 °C. At a working distance of 4.0 mm between the print head and the collector, MEW may be performed, for example with a voltage of 5.6 kV, print speed of 210 mm min$^{-1}$, and/or an air pressure of 2 bar for the macroporous patterns and 5.3 kV, 440 mm min$^{-1}$, and 0.5 bar for the microporous patterns.

All prints (MEW process) may be performed at a working distance, in particular in a range of 0.5 mm to 5 mm, more particularly in a range of 1 mm to 2.5 mm, for example at 2 mm. Prints may in particular be stabilized, for example for 8 min, by printing a sacrificial pattern before the actual scaffold fabrication. Parameters of the MEW process may include a pressure in a range of 0.1 bar to 2 bar, in particular in a range of 0.5 bar to 1.5 bar, for example 0.55 bar, 0.75 bar, 1.2 bar, or 1.3 bar. Additionally or alternatively, parameters of the MEW process may include a Voltage in a range of 2.5 kV to 3 kV, in particular in a range of 2.65 kV to 2.8 kV. Additionally or alternatively, parameters of the MEW process may include a print speed in a range of 250 mm/min to 2000 mm/min, in particular 300 mm/min to 1500 mm/min, more particularly 400 mm/min to 1200 mm/min or $600\pm25$ mm/min to $975\pm25$ mm/min. In a preferred embodiment, the MEW process may comprise a Voltage of 2.8 kV, a pressure of 1.5 bar, 0.8 bar, 0.5 bar or 0.1 bar and a print speed of 300 mm/min, 700 mm/min, 1200 mm/min, 1500 mmm/min or 1980 mm/min.

Compliance may be determined during the application of a pulsatile pressure profile (80 to 120 mmHg, $60 \pm 10$ pulses per minute) to the vascular graft device 1, thereby changing its diameter. The diameter of the vascular graft device 1 may be recorded, for example by the optical micrometer. The dynamic compliance may be determined, in particular according to ISO 7198:2016 (where p1 is the lower internal pressure and $r_{p1}$ the corresponding radius and $p_2$ is the higher internal pressure and $r_{p2}$ the corresponding radius):

$$C = \frac{(r_{p2} - r_{p1})/r_{p1}}{(p2 - p1)} * 10^4$$

Fig. 9 schematically illustrates a method 100 of manufacturing a vascular graft device 1 according to the further aspect of the present disclosure.

The method 100 comprises a step 200 of providing a mandrel 7.

After providing 200 the mandrel 7, the method comprises a step 300 of providing, in particular forming, a liner 3 on the mandrel 7. The liner 3 may be formed 300 in a melt electrowriting process. Forming 300 the liner 3 may be performed using a hemocompatible first polymer material and may in particular comprise forming a microporous fiber structure 30. By forming 300 the liner 3, a microporous scaffold is obtained. In embodiments wherein the liner 3 is provided directly on the mandrel 7, realizes a luminal scaffold.

The method 100 further comprises a step 400 of providing, in particular forming, a structural scaffold 5 surrounding and/or supporting the liner 3. Forming 400 the structural scaffold 5 may be performed using a biodegradable second polymer material and comprises forming a macroporous fiber structure 50. Thereby, a microporous scaffold may be obtained.

In some embodiments, step 400 may be performed before step 300, i.e., the structural scaffold 5 may be provided, in particular formed, directly on the mandrel 7. The microporous (fiber) structure 30/liner 3 may then be provided/formed surrounding the macroporous fiber structure 50. Step 300 and/or 400 may comprise performing a melt electrowriting process, respectively.

Additionally, the method 100 may comprise a step 500 of coating the liner 3 and/or the structural scaffold 5, with a hydrogel, such as a gelatine matrix. The vascular graft device 1 may for example be coated via a dip-coating process.

LIST OF REFERENCE SIGNS

1       vascular graft device
3       liner
5       structural scaffold
7       cylindrical mandrel
11      gradient section
13      first section (arterial)
15      second section (venous)
30      microporous fiber structure
50      macroporous fiber structure
91      vein
93      artery

100     method
200     providing/forming a mandrel
300     providing/forming a liner
400     providing/forming a structural scaffold
500     coating

D1, D2      graft diameter

$\alpha 1, \alpha 2$    fiber winding angle

**Claims**

1.  A vascular graft device (1), comprising

    a liner (3) comprising a microporous structure, in particular a microporous fiber structure (30), made from a first polymer material, wherein the first polymer material is hemocompatible; and
    a structural scaffold (5) supporting, in particular supporting and surrounding, the liner (3),
    wherein the structural scaffold (5) comprises a macroporous fiber structure (50), made from a second polymer material wherein the second polymer material is biodegradable.

2.  The vascular graft device (1) according to claim 1, wherein the macroporous fiber structure (50) is configured according to at least one predetermined structural parameter selected from the list comprising a fiber pattern, a fiber winding angle, a fiber spacing, a fiber diameter and a number of layers.

3.  The vascular graft device (1) according to claim 2, comprising at least one gradient section (11) extending in the lengthwise direction of the vascular graft device (1), wherein the at least one predetermined structural parameter changes continuously from a first value to a second value along a lengthwise direction of the vascular graft device (1) along the gradient section (11).

4.  The vascular graft device (1) according to claim 2 or 3, wherein the vascular graft device (1) is configured to be implanted at a host, wherein the at least one predetermined structural parameter is selected according to a mechanical compliance matching a characteristic mechanical compliance of a predetermined type of host vessel, such as an arterial host vessel or a venous host vessel.

5.  The vascular graft device (1) according to one of the claims 2 to 4, wherein the at least one predetermined structural parameter comprises a fiber winding angle in a range of 5° to 80°.

6.  The vascular graft device (1) according to claim 1, wherein the microporous fiber structure (30) is configured according to at least one second predetermined structural parameter selected from the list comprising a fiber pattern, a fiber winding angle, a fiber spacing, a fiber diameter and a number of layers.

7.  The vascular graft device (1) according to claim 6 and at least one of the claims 2 to 5, wherein the at least one predetermined structural parameter of the microporous fiber structure (30) differs from the at least one predetermined structural parameter of the macroporous fiber structure (50).

8.  The vascular graft device (1) according to at least one of the preceding claims, further comprising a hydrogel coating, in particular a gelatine coating.

9.  A method (100) of manufacturing a vascular graft device (1), comprising providing (200) a mandrel (7),

    forming (300) a liner (3) on the mandrel (7), wherein forming (300) the liner (3) is performed using a hemocompatible first polymer material and comprises forming a microporous structure, in particular a microporous fiber structure (30), and
    forming (400) a structural scaffold (5) for supporting, in particular for supporting and surrounding, the liner (3), wherein forming (400) the structural scaffold (5) is performed using a biodegradable second polymer material and comprises forming a macroporous fiber structure (50),
    wherein the macroporous fiber structure (50) is created in a melt electrowriting process,
    wherein optionally the microporous fiber structure (30) is also created in a melt electrowriting process.

10. The method (100) of manufacturing a vascular graft device (1) according to claim 9, wherein
    the macroporous fiber structure (50) is formed according to at least one predetermined structural parameter selected from the list comprising a fiber pattern, a fiber winding angle, a fiber spacing, a fiber diameter and a number of layers.

11. The method (100) of manufacturing a vascular graft device (1) according to claim 10, wherein the at least one predetermined structural parameter is changed continuously from a first value to a second value along a lengthwise direction of the vascular graft device during forming of the macroporous fiber structure (50) such that the vascular graft device (1) is provided with at least one gradient section (11) extending in the lengthwise direction of the vascular graft device (1).

12. The method (100) of manufacturing a vascular graft device (1) according to one of the claims 9 or 10, wherein the at least one predetermined structural parameter for forming the macroscopic fiber structure (50) is selected according to a mechanical compliance matching a characteristic mechanical compliance of a predetermined type of host vessel, such as an arterial host vessel or a venous host vessel.

13. The method (100) of manufacturing a vascular graft device (1) according to one of the claims 9 to 12, wherein a microporous fiber structure (30) is formed according to at least one predetermined structural parameter selected from the list comprising a fiber pattern, a fiber winding angle, a fiber spacing, a fiber diameter and a number of layers.

14. The method (100) of manufacturing a vascular graft device (1) according to claim 13 and at least one of the claims 10 to 12, wherein the at least one predetermined structural parameter of the microporous fiber structure (30) differs from the at least one predetermined structural parameter of the macroporous fiber structure (50).

15. The method (100) of manufacturing a vascular graft device (1) according to one of the claims 9 to 14, further comprising:
coating (500) the vascular graft device (1) with a hydrogel, in particular with gelatine.

_**Fig. 1a**_

_**Fig. 1b**_

_**Fig. 2**_

Fig. 3a   Fig. 3b   Fig. 3c   Fig. 3d   Fig. 3e   Fig. 3f

Fig. 4

Fig. 5

_Fig 6a_

_Fig. 6b_

_Fig. 6c_

_Fig. 6d_

Arterial Compliance   Gradient Compliance   Venous Compliance

_Fig. 6e_

_Fig. 6f_

_Fig. 6g_

*Fig. 7*

*Fig. 8*

*Fig. 9*

*Fig. 6h*

Europäisches
Patentamt

European
Patent Office

Office européen
des brevets

## EUROPEAN SEARCH REPORT

**Application Number**

EP 24 20 4797

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/003059 A1 (D'AMATO ANTHONY [US] ET AL) 4 January 2024 (2024-01-04)<br>* page 2, paragraph 0012 *<br>* page 4, paragraphs 0022, 0023, 0026, 0028 *<br>* example 3 *<br>* figure 12 *<br>* page 29, paragraphs 0246, 0247 *<br>----- | 1-7 | INV.<br>A61L27/18<br>A61L27/34<br>A61L27/50<br>A61L27/56<br>A61L27/58 |
| X | TOMASZ JUNGST ET AL: "Heterotypic Scaffold Design Orchestrates Primary Cell Organization and Phenotypes in Cocultured Small Diameter Vascular Grafts",<br>ADVANCED FUNCTIONAL MATERIALS, WILEY - V C H VERLAG GMBH & CO. KGAA, DE,<br>vol. 29, no. 43,<br>16 August 2019 (2019-08-16), page n/a,<br>XP072408030,<br>ISSN: 1616-301X, DOI:<br>10.1002/ADFM.201905987 | 1,2,<br>4-10,<br>12-15 | |
| Y | * abstract *<br>* page 2, paragraph 2.2. Results and Discussion *<br>----- | 11 | **TECHNICAL FIELDS SEARCHED (IPC)**<br><br>A61L |
| Y | MCCOSKER AUDREY B. ET AL: "Exploiting Nonlinear Fiber Patterning to Control Tubular Scaffold Mechanical Behavior",<br>ADVANCED MATERIALS TECHNOLOGIES,<br>vol. 7, no. 11, 13 July 2022 (2022-07-13),<br>XP093164970,<br>ISSN: 2365-709X, DOI:<br>10.1002/admt.202200259<br>Retrieved from the Internet:<br>URL:https://onlinelibrary.wiley.com/doi/full-xml/10.1002/admt.202200259><br>* abstract *<br>* page 3, paragraph 2.4. Heterogeneous Scaffold Design *<br>-----<br>-/-- | 11 | |

The present search report has been drawn up for all claims

| Place of search | Date of completion of the search | Examiner |
|---|---|---|
| The Hague | 11 March 2025 | Dudás, Eszter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 .............................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

**EP 24 20 4797**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PIEN NELE ET AL: "Polymeric reinforcements for cellularized collagen-based vascular wall models: influence of the scaffold architecture on the mechanical and biological properties", FRONTIERS IN BIOENGINEERING AND BIOTECHNOLOGY, vol. 11, 16 November 2023 (2023-11-16), XP093258176, CH ISSN: 2296-4185, DOI: 10.3389/fbioe.2023.1285565 * abstract * * page 5, paragraph 2.6. * * page 15, paragraph 4. * ----- | 1-15 | |

**TECHNICAL FIELDS SEARCHED (IPC)**

The present search report has been drawn up for all claims

| Place of search | Date of completion of the search | Examiner |
|---|---|---|
| The Hague | 11 March 2025 | Dudás, Eszter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 721 770 A1

## ANNEX TO THE EUROPEAN SEARCH REPORT
## ON EUROPEAN PATENT APPLICATION NO.

EP 24 20 4797

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2025

| Patent document cited in search report | Publication date | Patent family member(s) | | Publication date |
|---|---|---|---|---|
| US 2024003059 A1 | 04-01-2024 | US | 2024003059 A1 | 04-01-2024 |
| | | WO | 2022087487 A1 | 28-04-2022 |

EPO FORM P0459

For more details about this annex : see Official Journal of the European Patent Office, No. 12/82

23

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220257371 A1